# EUROPEAN PATENT SPECIFICATION

(11) **EP 2 136 784 B1**
(45) Date of publication and mention of the grant of the patent: **04.11.2015**
(21) Application number: 08726058.4
(22) Date of filing: 26.02.2008
(51) Int. Cl.: A61K 9/107, A61K 31/20

(54) **BIO-ABSORBABLE OIL SUSPENSION**
BIOLOGISCH RESORBIERBARE ÖLSUSPENSION
SUSPENSION D'HUILE BIOABSORBABLE

(30) Priority: 27.02.2007 US 711389
(43) Date of publication of application: 30.12.2009
(73) Proprietor: Atrium Medical Corporation, Hudson, NH 03051 (US)
(72) Inventor: HERWECK, Steve, A., Nashua, NH 03062 (US); KARWOSKI, Theodore, Hollis, NH 03049 (US); MARTAKOS, Paul, Pelham, NH 03076 (US)
(74) Representative: Helbig, Christian
(86) International application number: PCT/US2008/002474
(87) International publication number: WO 2008/106094

(56) References cited:
- WO-A1-90/08544
- WO-A1-99/08544
- US-A- 4 937 254
- US-A- 5 411 988
- US-A1- 2005 186 244
- US-A1- 2006 088 596
- AGIR ET AL: "A novel method to prevent tissue desiccation during burn surgery: Saline spray", BURNS, BUTTERWORTH HEINEMANN, GB, vol. 33, no. 6, 1 September 2007 (2007-09-01), pages 804-805, XP022202588, ISSN: 0305-4179, DOI: 10.1016/J.BURNS.2007.01.008

## Description

### FIELD OF THE INVENTION

The present invention relates to a dispersing liquid for local application to internal body tissues and cavities, a method of making said liquid, methods of using said liquid, and a kit including said liquid. More specifically, the present invention is directed to a dispersing liquid for coating internal body tissues with a thin layer of bio-absorbable oil. The dispersing liquid includes a suspension of bio-absorbable oil in a liquid carrier and can optionally include one or more locally applied therapeutic agents, analgesics, and imaging enhancing agents.

### BACKGROUND OF THE INVENTION

During surgery, substantial cell and tissue damage can be caused by desiccation of tissue due to exposure to air and unwanted adhesion between different tissues and between tissues and implants or instruments Surgical intervention can cause desiccational injuries when normally wet internal or moist body cavity tissues (including abdominal, nasal, sinus, vaginal, etc.) dry out after prolonged exposure to air or other gases. Desiccational injuries can result in inflammation, dense connective tissue remodeling, adhesion formation, and pain and localized inflammation of other adjacent tissues. Additionally, other operative tissue injuries during surgery, such as heat ablation, injuries resulting from penetrating metal tissue fixation, tissue compression, and tissue exposure to positive pressure insufflation, can cause various forms of unwanted connective tissue adhesion formation between body cavity tissues, membranes, organs, and/or tissue adhesions to implanted fixation and tissue reinforcing medical devices. Minimizing desiccation of tissue and providing a lubricant to prevent tissue adhesion could reduce pain, inflammation, and connective tissue remodeling associated with some surgeries.

Suspensions of bio-absorbable oil with emulsifier in a liquid carrier are known for rinsing tissues and preventing tissue adhesion after surgery, as for example disclosed in US5411988, US4937254 and WO90/08544. Saline sprays without oil are known in the prevention of tissue dessication during surgery, see H. Agir et al in Burns 33 (2007) 804-805.

Ideally, a dispersing liquid could be applied to internal body tissues during surgery that would minimize exposure of wet tissues to air preventing desiccational injuries and that would act as a therapeutic lubricant to reduce adhesion formation between various tissues and between tissues, surgical tools and implants. The dispersing liquid itself should adhere to tissues and form a complete and uniform thin coating covering the exposed tissues. Additionally, the dispersing liquid could contain therapeutic agents to reduce scar formation, prevent infection, etc. and analgesic agents.

### SUMMARY OF THE INVENTION

In accordance with the present invention, a dispersing liquid, a method of making a dispersing liquid, and a kit including a dispersing liquid are provided that prevent tissue desiccation, provide therapeutic lubrication, prevent unwanted tissue adhesion, and reduce inflammation due to tissue trauma.

The present invention, as defined by the claims, includes a dispersing liquid for distributing a bio-absorbable oil to internal body tissues. The dispersing liquid includes a suspension formulated for local application to internal body tissues. The suspension includes a liquid carrier and a bio-absorbable oil suspended in the liquid carrier. According to aspects of the invention, the bio-absorbable oil can include at least one form of lipid. The bio-absorbable oil can include at least one form of fatty acid. The bio-absorbable oil can include one or both of a synthetic bio-absorbable oil or a naturally occurring bio-absorbable oil. The bio-absorbable oil can include a combination of a plurality of types of bio-absorbable oils. The bio-absorbable oil can include a partially cured liquid bio-absorbable oil. The liquid carrier can include any of water, an alcohol, a solution of dextrose in water, a sodium phosphate solution, a hydrochloric acid solution, and a saline solution.

The bio-absorbable oil is configured to adhere to internal body tissues upon application of the suspension. The suspension is configured to result in a thin coating of bio-absorbable oil on surfaces of the internal body tissues upon application of the suspension to the internal body tissues. The suspension is configured to result in the bio-absorbable oil adhering to surfaces of the internal body tissues and coalescing into a thin film coating on surfaces of the internal body tissues upon application.

According to still other aspects of the invention, the dispersing liquid can include a therapeutic agent. The therapeutic agent can be in the form of a solid suspended in the liquid carrier, a solid suspended in the bio-absorbable oil, or both. The dispersing liquid can include one or more of an anti-proliferative agent, an anti-inflammatory agent, or an anti-infective agent. The dispersing liquid can include a local analgesic agent. The dispersing liquid can include one or both of an imaging enhancing agent or a visibly detectable color additive.

The liquid carrier and the bio-absorbable oil form an unstable suspension of bio-absorbable oil suspended in the liquid carrier formulated for local application to internal body tissues.

The invention includes a method for making a dispersing liquid. The method includes providing a bio-absorbable oil, providing a liquid carrier, and mixing the bio-absorbable oil and the liquid carrier to form a suspension of liquid bio-absorbable oil suspended in the liquid carrier and formulated for local application to internal body tissues. The suspension is not stabilized.

Another illustrative aspect is a method of preventing tissue desiccation during surgery. The method includes providing a dispersing liquid formulated for preventing tissue desiccation. The suspension includes a liquid carrier and a bio-absorbable oil suspended in the liquid carrier. The method also includes locally applying the dispersing liquid to internal body tissues forming a thin coating of bio-absorbable oil on the internal body tissues. According to aspects of the invention, the method can also include reapplying the dispersing liquid to internal body tissues. The method can further include removing an excess liquid from a body cavity containing the internal body tissues, the excess liquid comprising the liquid carrier. When the excess liquid is removed the thin coating of bio-absorbable oil can remain on the internal body tissues.

Yet another aspect is a method of treating tissue desiccation during surgery. The method includes providing a dispersing liquid formulated for treating tissue desiccation. The dispersing liquid includes a suspension including a liquid carrier and a bio-absorbable oil suspended in the liquid carrier. The method further includes locally applying the dispersing liquid to internal body tissues forming a thin coating of bio-absorbable oil on the internal body tissues.

Still another aspect is a method of providing therapeutic lubrication to tissues and organs during and after surgery. The method includes providing a dispersing liquid formulated for therapeutic lubrication. The dispersing liquid includes a suspension including a liquid carrier and a bio-absorbable oil suspended in the liquid carrier. The method further includes locally applying the dispersing liquid to internal body tissues forming a thin coating of bio-absorbable oil on the internal body tissues.

Another aspect is a method of providing therapeutic lubrication to medical devices adapted to come into contact with tissues and organs during surgery. The method includes providing a dispersing liquid formulated for therapeutic lubrication. The dispersing liquid includes a suspension including a liquid carrier and a bio-absorbable oil suspended in the liquid carrier. The method further includes locally applying the dispersing liquid to internal body tissues and instruments that come in contact with tissues and organs during surgery forming a thin coating of bio-absorbable oil on the internal body tissues and instruments.

Yet another aspect is a method of reducing inflammation due to tissue trauma. The method includes providing a dispersing liquid formulated for reducing inflammation. The dispersing liquid includes a suspension including a liquid carrier and a bio-absorbable oil suspended in the liquid carrier. The method also includes locally applying the dispersing liquid to internal body tissues forming a thin coating of bio-absorbable oil on the internal body tissues.

The invention also includes a kit for coating internal body tissues with a coating suspension. The kit includes a liquid carrier formulated for local application to internal body tissues and a bio-absorbable oil formulated for local application to internal body tissues. According to aspects of the invention, the kit can also include a sterile measured mixing apparatus. The sterile measured mixing apparatus can be an applicator. The kit can also include packaging to maintain the sterility of the measured mixing apparatus. The kit can also include an applicator.

### BRIEF DESCRIPTION OF THE DRAWINGS

The aforementioned features and advantages, and other features and aspects of the present invention, will become better understood with regard to the following description and accompanying drawings, wherein:
**FIG. 1A** is a diagrammatic illustration of a dispersing liquid comprising a suspension in a syringe according to one embodiment of the present invention;
**FIG. 1B** is a diagrammatic illustration of a dispersing liquid comprising a suspension in a measured mixing apparatus according to one embodiment of the present invention;
**FIG. 2** is a flow chart illustrating a method of making a dispersing liquid according to another embodiment of the present invention;
**FIG. 3A** is a diagrammatic illustration of two immiscible liquids in the form of separated layers;
**FIG. 3B** is a diagrammatic illustration of two immiscible liquids in the form of an emulsion;
**FIG. 4A** is a flow chart illustrating a method of preventing tissue desiccation during surgery using a dispersing liquid according to another embodiment of the present invention;
**FIG. 4B** is a flow chart illustrating a method of treating tissue desiccation during surgery using a dispersing liquid according to aspects of the present invention;
**FIG. 4C** is a flow chart illustrating a method of providing therapeutic lubrication to tissues and organs during and after surgery using a dispersing liquid according to aspects of the present invention;
**FIG. 4D** is a flow chart illustrating a method of providing therapeutic lubrication to medical devices adapted to come into contact with tissues and organs during surgery using a dispersing liquid according to aspects of the present invention;
**FIG. 4E** is a flow chart illustrating a method of preventing inflammation using a dispersing liquid according to aspects of the present invention;
**FIG. 5** is a diagrammatic illustration of application of the dispersing liquid to internal body tissues during surgery;
**FIG. 6A** is a diagrammatic illustration of the dispersing liquid shortly after application to internal body tissues;
**FIG. 6B** is a diagrammatic illustration of the dispersing liquid as the droplets of bio-absorbable oil begin to adhere and coalesce after application to internal body tissues;
**FIG. 6C** is a diagrammatic illustration of the bio-absorbable oil coating on internal body tissues;
**FIG. 7A** is a diagrammatic illustration of a bio-absorbable oil coating on internal body tissues that does not include a visibly detectable color additive or an imaging enhancing agent;
**FIG. 7B** is a diagrammatic illustration of a bio-absorbable oil coating on internal body tissues that includes a visibly detectable color additive;
**FIG. 7C** is a diagrammatic illustration of a bio-absorbable oil coating on internal body tissues that includes an imaging enhancing agent; and
**FIG. 8** is a diagrammatic illustration of a kit including a dispersing liquid according to another embodiment of the present invention.

### DETAILED DESCRIPTION

Illustrative embodiments of the present invention include a dispersing liquid having a suspension of bio-absorbable oil suspended in a liquid carrier for distributing a bio-absorbable oil to internal body tissues, a method of making said dispersing liquid, and a kit for coating internal body tissues with said dispersing liquid. A dispersing liquid is locally applied to internal body tissues forming a thin coating of bio-absorbable oil on the internal body tissues. As the liquid carrier of the applied dispersing liquid spreads over the internal body tissues, droplets of the bio-absorbable oil suspended in the liquid carrier are spread with it. The droplets of oil adhere to the internal body tissues and coalesce forming a thin coating of bio-absorbable oil at least partially covering the tissues. This thin coating of bio-absorbable oil prevents and treats tissue desiccation, and provides therapeutic lubrication to tissues, organs, and medical devices adapted to come into contact with tissues and organs during and after surgery, thereby preventing unwanted tissue adhesion and reducing inflammation due to tissue trauma.

As utilized herein, the term "bio-absorbable" generally refers to having the property or characteristic of being able to penetrate the tissue cells of a patient's body. In certain embodiments of the present invention bio-absorption occurs through a lipophilic mechanism. The bio-absorbable oil is soluble in the phospholipid bi-layer of cells of body tissue and therefore impacts how the bio-absorbable substance penetrates into the cells.

It should be noted that a bio-absorbable substance is different from a biodegradable substance. Biodegradable is generally defined as capable of being decomposed by biological agents, or capable of being broken down by microorganisms or biological processes, in a manner that does not result in cellular uptake of the biodegradable substance. Biodegradation thus relates to the breaking down and distributing of a substance through the patient's body, versus the substance penetrating of the cells of the patient's body tissue. Biodegradable substances can cause inflammatory response due to either the parent substance or substances formed during breakdown. The parent substance and substances formed during breakdown may or may not be absorbed by tissues.

With regard to bio-absorbable oils, it is generally known that the greater the degree of unsaturation of fatty acids of a fat the lower the melting point of the fat, and the longer the hydrocarbon chains of a fat the higher the melting point of the fat. A polyunsaturated fat, thus, has a lower melting point, and a saturated fat has a higher melting point. Those fats having a lower melting point are more often oils at room temperature. Those fats having a higher melting point are more often waxes or solids at room temperature. Therefore, a fat having the physical state of a liquid at room temperature is an oil. In general, polyunsaturated fats are liquid oils at room temperature, and saturated fats are waxes or solids at room temperature.

Polyunsaturated fats are one of four basic types of fat derived by the body from food. The other fats include saturated fat, as well as monounsaturated fat and cholesterol. Polyunsaturated fats can be further composed of omega-3 fatty acids and omega-6 fatty acids. Under the convention of naming the unsaturated fatty acid according to the position of its first double bond of carbons, those fatty acids having their first double bond at the third carbon atom from the methyl end of the molecule are referred to as omega-3 fatty acids. Likewise, a first double bond at the sixth carbon atom is called an omega-6 fatty acid. There can be both monounsaturated and polyunsaturated omega fatty acids.

Omega-3 and omega-6 fatty acids are also known as essential fatty acids because they are important for maintaining good health, despite the fact that the human body cannot make them on its own. As such, omega-3 and omega-6 fatty acids must be obtained from external sources, such as food. Omega-3 fatty acids can be further characterized as containing eicosapentaenoic acid (EPA), docosahexanoic acid (DHA), and alpha-linolenic acid (ALA). Both EPA and DHA are known to have anti-inflammatory effects and wound healing effects within the human body.

As utilized herein, a suspension is a mixture in which a finely-divided species is distributed throughout a different continuous species, where the finely-divided species is distributed by the introduction of mechanical energy, by stirring, agitation, etc. A suspension, in which the introduction of mechanical energy distributes the finely-divided species, should be distinguished from a solution in which entropy maintains the distribution of one species throughout another. Some narrow definitions of a suspension specify that the finely-divided species is a solid, however, as utilized herein, the more general definition of suspension is employed in which the finely-divided species of a suspension may be a liquid, a gel, or a solid. If the finely-divided species is a liquid and the continuous species is a liquid, then the suspension may also be described as an emulsion. If the suspension is an emulsion, then the introduction of mechanical energy both creates the finely-divided species (by breaking one species up into finely-divided droplets) and distributes the finely-divided species. As utilized herein, a suspension may consist of solid particles distributed in a continuous liquid species, liquid droplets distributed in a continuous liquid species, gel particles distributed in a continuous liquid species, or any combination of solid particles, gel particles and liquid droplets distributed in a liquid species. Additionally, liquid droplets in a suspension may also have solid particles suspended within them. A suspension may include a finely divided liquid species and one or more finely divided solid or gel species distributed throughout the continuous species.

Some other narrow definitions of a suspension specify a particular size range for the diameter of the finely-divided species, however, as utilized herein, the diameter of finely-divided species in the suspension is limited only in that it must be small enough such that the finely-divided species does not substantially coalesce, separate or settle on the relevant working time scale.

An "emulsion," which is a type of suspension, is a combination of two or more immiscible liquids in a particular energetically unstable state. Although an emulsion can be a combination of more than two immiscible liquids, for the sake of clarity, the following explanation will be presented assuming an emulsion of only two liquids. The first liquid is dispersed or suspended in a continuous phase of the second liquid. This may be thought of as "droplets" of the first suspended liquid distributed throughout a continuous "pool" of the second liquid. The first liquid can include a mixture of any number of miscible liquids and the second liquid can include a mixture of any number of miscible liquids as long as the mixture of the first liquid is immiscible with respect to the mixture of the second liquid. One of ordinary skill in the art will appreciate that emulsions can be formulated using a combination of three or more immiscible liquids, and although such embodiments are not described further herein, they are considered to fall within the scope of the present invention.

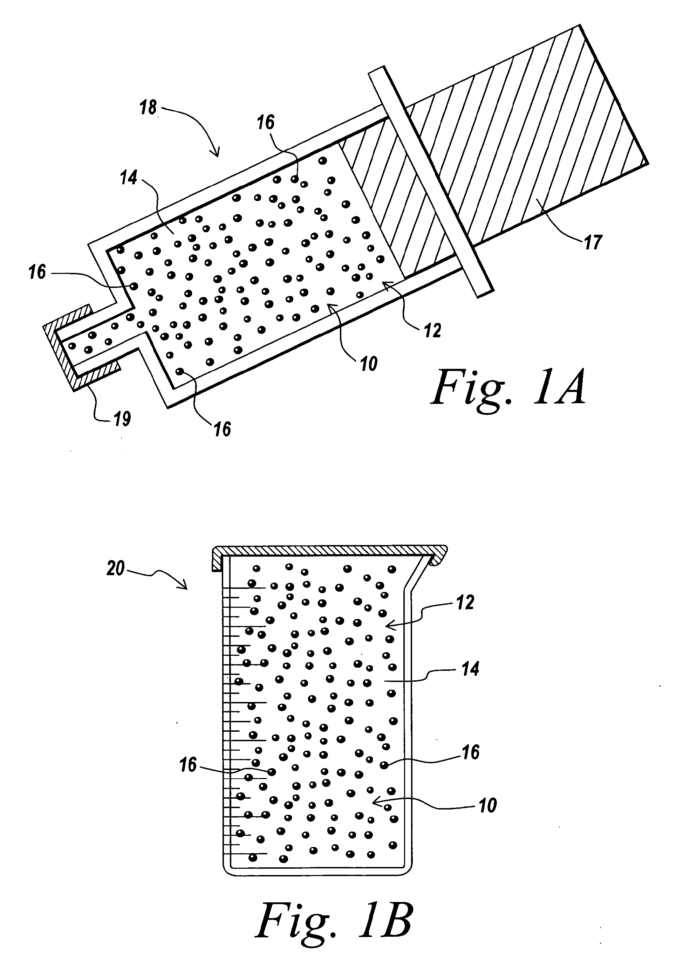

**FIGS. 1A** through 8, wherein like parts are designated by like reference numerals throughout, illustrate example embodiments of a dispersing liquid including a suspension of bio-absorbable oil suspended in a liquid carrier, a method of making the dispersing liquid, methods of using the dispersing liquid to prevent tissue desiccation, treat tissue desiccation, provide therapeutic lubrication, prevent unwanted tissue adhesion, and reduce inflammation, and a kit for coating internal body tissues with a dispersing liquid.

**FIG. 1A** depicts a dispersing liquid 10 according to an exemplary embodiment of the invention. The dispersing liquid 10 includes a suspension 12 that is formulated for local application to internal body tissues. The suspension is formulated with at least a liquid carrier 14 and a liquid bio-absorbable oil 16 suspended in the liquid carrier. In this embodiment, the dispersing liquid 10 is contained in a syringe 18 that can be used to apply the dispersing liquid 10 to internal body tissues. The syringe 18 includes a cap 19 for preventing oxygen from entering the syringe 18 and to contain the dispersing liquid 10 within the syringe 18. The dispersing liquid 10 can be dispensed by removing the cap 19 and depressing a plunger 17.

**FIG. 1B** depicts the dispersing liquid 10 that includes a suspension 12 of bio-absorbable oil 16 dispersed in a liquid carrier 14 that is contained in a measured mixing apparatus 20. The measured mixing apparatus 20 is used to agitate and form the suspension 12 and can also be used to apply the suspension 12 to a desired tissue location.

For the dispersing liquid to be suitable for local application to internal body tissues, the bio-absorbable oil and the liquid carrier must be formulated to be biocompatible. In this context biocompatible means non-toxic, non-allergenic, non-mutagenic, non-carcinogenic and non-inflammatory. Additionally, liquids formulated for local application to internal body tissues must be sterile. Also, liquids formulated for local application to internal body tissues must be formulated for absorption and/or excretion by the body.

In the suspension 12 of an illustrative embodiment, the liquid carrier 14 is the continuous component of the suspension 12 and the bio-absorbable oil 16 is the suspended component of the suspension 12. Thus, "droplets" of bio-absorbable oil 16 are suspended in the liquid carrier 14 and distributed throughout the liquid carrier 14. In **FIG. 1A** and **FIG. 1B** the size of the "droplets" of bio-absorbable oil 16 is greatly exaggerated for illustrative purposes

Bio-absorbable oil is non-toxic, non-allergenic, non-mutagenic and non-inflammatory. Additionally bio-absorbable oil can be sterilized. The bio-absorbable oil 16 can include any of a synthetic bio-absorbable oil and a naturally occurring bio-absorbable oil. Examples of naturally occurring bio-absorbable oils are fish oil, cod liver oil, squid oil and plant derived oils. A characteristic of a naturally occurring oil is that the oil includes lipids. In addition, a naturally occurring oil includes omega-3 fatty acids in accordance with several embodiments of the present invention. As previously described, omega-3 fatty acids and omega-6 fatty acids are known as essential fatty acids. Omega-3 fatty acids can be further characterized as eicosapentaenoic acid (EPA), docosahexanoic acid (DHA), and alpha-linolenic acid (ALA). Both EPA and DHA are known to have anti-inflammatory effects and wound healing effects within the human body.

The liquid carrier 14 is bio-absorbable and has an appropriate viscosity or liquidity such that, when mixed with the bio-absorbable oil 6, the resulting suspension 12 can be easily applied over a large surface area of internal body tissues. The liquid carrier 14 can be any of water, an alcohol, a solution of dextrose in water, a sodium phosphate solution, a hydrochloric acid solution, and a saline solution which are all known to be suitable for local application to internal body tissues. Additionally, one of skill in the art will appreciate that the liquid carrier 14 could be a combination of any of the aforementioned liquids or other suitable liquids having equivalent physical and chemical properties.

The suspension 12 can also contain one or more therapeutic agents. The therapeutic agent can be mixed with the bio-absorbable oil 16 and the liquid carrier 14 to form the suspension 12. The therapeutic agent may dissolve or mix to form a solution with the bio-absorbable oil 16. The therapeutic agent may instead be a solid or a liquid suspended in either the liquid carrier 14 or the bio-absorbable oil. The therapeutic agent can take a number of different forms including but not limited to anti-oxidants, anti-inflammatory agents, anti-coagulant agents, drugs to alter lipid metabolism, antiproliferatives, anti-neoplastics, tissue growth stimulants, functional protein/factor delivery agents, anti-infective agents, imaging agents, anesthetic agents, therapeutic agents, tissue absorption enhancers, anti-adhesion agents, germicides, antiseptics, proteoglycans, GAG's, gene delivery (polynucleotides), polysaccharides (e.g., heparin), anti-migratory agents, pro-healing agents, ECM/protein production inhibitors, analgesics, prodrugs, and any additional desired therapeutic agents such as those listed in Table 1 below.

**TABLE 1**

| **CLASS** | **EXAMPLES** |
|---|---|
| Antioxidants | Alpha-tocopherol, lazaroid, probucol, phenolic antioxidant, resveretrol, AGI-1067, vitamin E |
| Antihypertensive Agents | Diltiazem, nifedipine, verapamil |
| Antiinflammatory Agents | Glucocorticoids (e.g. dexamethazone, methylprednisolone), leflunomide, NSAIDS, ibuprofen, acetaminophen, hydrocortizone acetate, hydrocortizone sodium phosphate, macrophage-targeted bisphosphonates, pimecrolimus, ISA247 |
| Growth Factor Antagonists | Angiopeptin, trapidil, suramin |
| Antiplatelet Agents | Aspirin, dipyridamole, ticlopidine, clopidogrel, GP IIb/IIIa inhibitors, abcximab |
| Anticoagulant Agents | Bivalirudin, heparin (low molecular weight and unfractionated), wafarin, hirudin, enoxaparin, citrate |
| Thrombolytic Agents | Alteplase, reteplase, streptase, urokinase, TPA, citrate, tenectaplase |
| Drugs to Alter Lipid Metabolism (e.g. statins) | Fluvastatin, colestipol, lovastatin, atorvastatin, amlopidine |
| ACE Inhibitors | Elanapril, fosinopril, cilazapril |
| Antihypertensive Agents | Prazosin, doxazosin |
| Antiproliferatives and Antineoplastics | Cyclosporine, cochicine, mitomycin C, sirolimus micophenonolic acid, rapamycin, everolimus, tacrolimus, paclitaxel, QP-2, actinomycin, estradiols, dexamethasone, methatrexate, cilostazol, prednisone, cyclosporine, doxorubicin, ranpirnas, troglitzon, valsarten, pemirolast, C-MYC antisense, angiopeptin, vincristine, PCNA ribozyme, 2-chloro-deoxyadenosine, mTOR targeting compounds |
| Tissue growth stimulants | Bone morphogeneic protein, fibroblast growth factor, SAR943, TAFA93 |
| Promotion of hollow organ occlusion or thrombosis | Alcohol, surgical sealant polymers, polyvinyl particles, 2-octyl cyanoacrylate, hydrogels, collagen, liposomes |
| Functional Protein/Factor delivery | Insulin, human growth hormone, estradiols, nitric oxide, endothelial progenitor cell antibodies |
| Second messenger targeting | Protein kinase inhibitors |
| Angiogenic | Angiopoetin, VEGF |
| Anti-Angiogenic | Endostatin |
| Inhibition of Protein Synthesis/ECM formation | Halofuginone, prolyl hydroxylase inhibitors, C-proteinase inhibitors |
| Antiinfective Agents | Penicillin, gentamycin, adriamycin, cefazolin, amikacin, ceftazidime, tobramycin, levofloxacin, silver, copper, hydroxyapatite, vancomycin, ciprofloxacin, rifampin, mupirocin, RIP, kanamycin, brominated furonone, algae byproducts, bacitracin, oxacillin, nafcillin, floxacillin, clindamycin, cephradin, neomycin, methicillin, oxytetracycline hydrochloride, Selenium, cefipime, metronidazole |
| Gene Delivery | Genes for nitric oxide synthase, human growth hormone, antisense oligonucleotides |
| Local Tissue perfusion | Alcohol, H₂O, saline, fish oils, vegetable oils, liposomes |
| Nitric oxide Donor Derivatives | NCX 4016 - nitric oxide donor derivative of aspirin, SNAP |
| Gases | Nitric oxide, compound solutions |
| Imaging Agents | Halogenated xanthenes, diatrizoate meglumine, diatrizoate sodium, biomarker agents |
| Anesthetic Agents | Lidocaine, benzocaine |
| Descaling Agents | Nitric acid, acetic acid, hypochlorite |
| Anti-Fibrotic Agents | Interferon gamma -1b, Interluekin - 10 |
| Immunosuppressive/Immu nomodulatory Agents | Cyclosporine, rapamycin, mycophenolate motefil, leflunomide, tacrolimus, tranilast, interferon gamma-1b, mizoribine, mTOR targeting compounds |
| Chemotherapeutic Agents | Doxorubicin, paclitaxel, tacrolimus, sirolimus, fludarabine, ranpirnase |
| Tissue Absorption Enhancers | Fish oil, squid oil, omega 3 fatty acids, vegetable oils, lipophilic and hydrophilic solutions suitable for enhancing medication tissue absorption, distribution and permeation |
| Anti-Adhesion Agents | Hyaluronic acid, human plasma derived surgical sealants, and agents comprised of hyaluronate and carboxymethylcellulose that are combined with dimethylaminopropyl, ethylcarbodimide, hydrochloride, PLA, PLGA |
| Ribonucleases | Ranpirnase |
| Germicides | Betadine, iodine, sliver nitrate, furan derivatives, nitrofurazone, benzalkonium chloride, benzoic acid, salicylic acid, hypochlorites, peroxides, thiosulfates, salicylanilide |
| Antiseptics | Selenium |
| Analgesics | Bupivicaine, naproxen, ibuprofen, acetylsalicylic acid |

Some specific examples of useful therapeutic agents include local analgesic compounds, such as Bupivacaine HCL (Marcaine^{®}), which reduce localized body cavity pain, and anti-inflammatory agents, such as Sirolimus (Rapamycin) and ISA 247 (Calcinuerin inhibiting compound) or Dexamethasone sodium phosphate (Glucocorticosteroid), which reduce inflammation or swelling following tissue injury. Anti-infective agents, such as rifampin, vancomycin, and gentamycin, may prevent infection following surgery. Additionally, an antiproliferative agent, such as rapamycin, reduces cellular remodeling and helps prevent connective tissue scar formation.

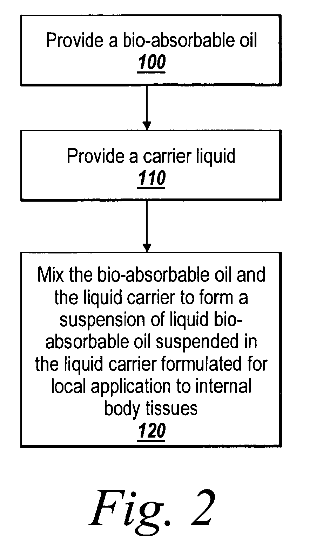

**FIG. 2** illustrates a method of making a dispersing liquid according to an exemplary embodiment of the invention. The process involves providing a bio-absorbable oil 16 (step 100) and providing a liquid carrier 14 (step 110). The bio-absorbable oil 16 and the liquid carrier 14 are mixed to form a suspension 12 of bio-absorbable oil 16 suspended in the liquid carrier 14 formulated for local application to internal body tissues (step 120). The suspension is not stabilized.

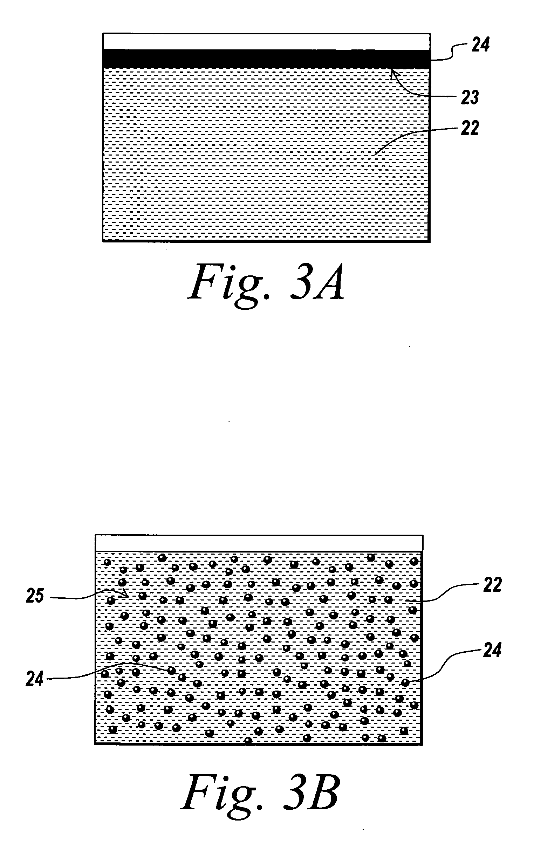

A suspension is an energetically unstable state. An emulsion, being a form of a suspension, is also an energetically unstable state. FIG. 3A shows the energetically stable state of a combination of two immiscible liquids, which is two separate layers of liquid; one layer of the first (more dense) liquid 22 below another layer of the second (less dense) liquid 24. A configuration of two separate layers of liquid , as shown in **FIG. 3A****,** is energetically stable because the configuration minimizes the area of a high energy boundary interface 23 between the first liquid 22 and the second liquid 24.

Because an emulsion is an energetically unstable state, simply pouring a first liquid 22 and a second liquid 24, which are immiscible, into the same container will not create an emulsion. Energy must be introduced to the system in order to form an emulsion. The energy may be introduced by agitating, mixing, or shaking the combination of the two immiscible liquids to form an emulsion. Other known methodologies for forming an emulsion also fall within the scope of the invention. FIG. 3B depicts an emulsion 25 of the second liquid 24 suspended in the first liquid 22. The size of the droplets of the suspended second liquid 24 is greatly exaggerated for illustrative purposes. In general, the size (diameter) of the droplets in the emulsion is inversely related to the energy used to agitate the mixture forming the emulsion. Usually, the greater the agitation energy, the smaller the droplets of the second liquid 24 suspended in the first liquid 22. Because an emulsion is an unstable state, as soon as the introduction of energy in the form of mixing or shaking ceases the two liquids will begin returning to their stable state. One way that an emulsion returns to its stable state is through coalescence. During coalescence "droplets" of the suspended phase combine to form bigger "droplets". An emulsion is "broken" or "separated" when sufficient coalescence has taken place such that that there are layers of liquid instead of droplets of one liquid suspended in the other. After "break" or "separation" the two liquids are no longer an emulsion.

Although an emulsion is an inherently energetically unstable state, an emulsion can be "stabilized" so that it will not "break" or "separate" within a relevant working time scale. Although a "stabilized" emulsion is energetically unstable, it is described as a stable emulsion because it is stable for practical purposes on the relevant working time scale. In general, emulsions can be stabilized so that they will require years to separate or break. Agents that stabilize emulsions are called stabilizers or emulsifiers. Examples of physiologically safe emulsifiers used for oil-in-water emulsions are phospholipids (like ovolecithin) and proteins.

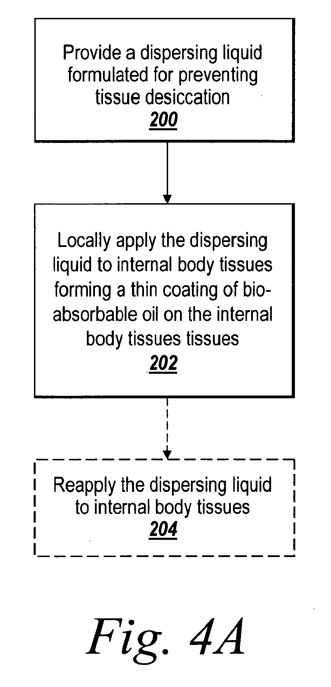

**FIG. 4A** illustrates a method of preventing tissue desiccation during surgery. Initially, a dispersing liquid 10 formulated for preventing tissue desiccation is provided (step 200). The dispersing liquid 10 includes a suspension 12 including a liquid carrier 14 and a bio-absorbable oil 16 suspended in the liquid carrier 14. The dispersing liquid is locally applied to internal body tissues forming a thin coating of bio-absorbable oil on the internal body tissues (step 202).

The dispersing liquid is a suspension 12 with the bio-absorbable oil 16 suspended in the liquid carrier 14. Because the suspension 12 would eventually separate into layers of component liquids, the dispersing liquid can be shaken or agitated shortly before being applied to the internal body tissues to ensure that the dispersing liquid 10 is in the form of a suspension 12 when it is applied. Some commonly used emulsifiers and stabilizers used to "stabilize" a suspension in the form of an emulsion also prevent droplets in the suspension from adhering to internal body tissues. The suspensions which are embodiments of the present invention cannot contain an agent or component that would prevent the droplets of bio-absorbable oil from adhering to internal body tissues.

The dispersing liquid can be applied by numerous mechanical means including a liquid spray apparatus, aerosol fogging, a syringe, a remote or directly applied catheter injection, a hollow needle and/or a trocar insertion. Additionally, the dispersing liquid can be applied by direct tissue splash, rise and/or wash.

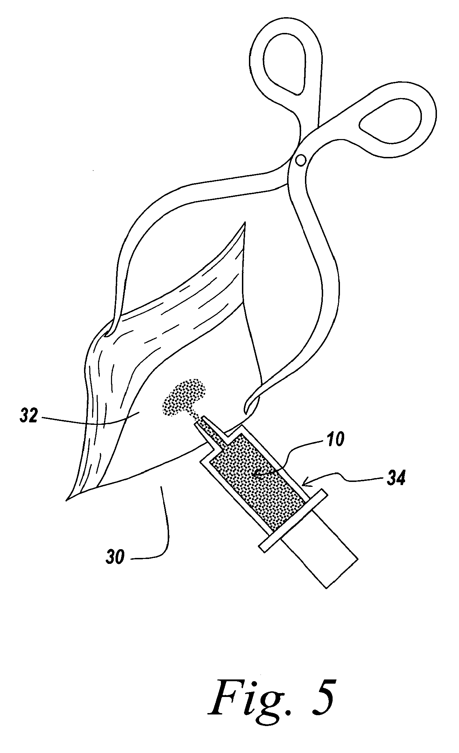

**FIG. 5** illustrates application of the dispersing liquid to internal body tissues. During surgery external tissue 30 may be cut exposing internal body tissues 32. The dispersing liquid 10 is applied to the internal body tissues 32 using a syringe 34 in accordance with one example application embodiment.

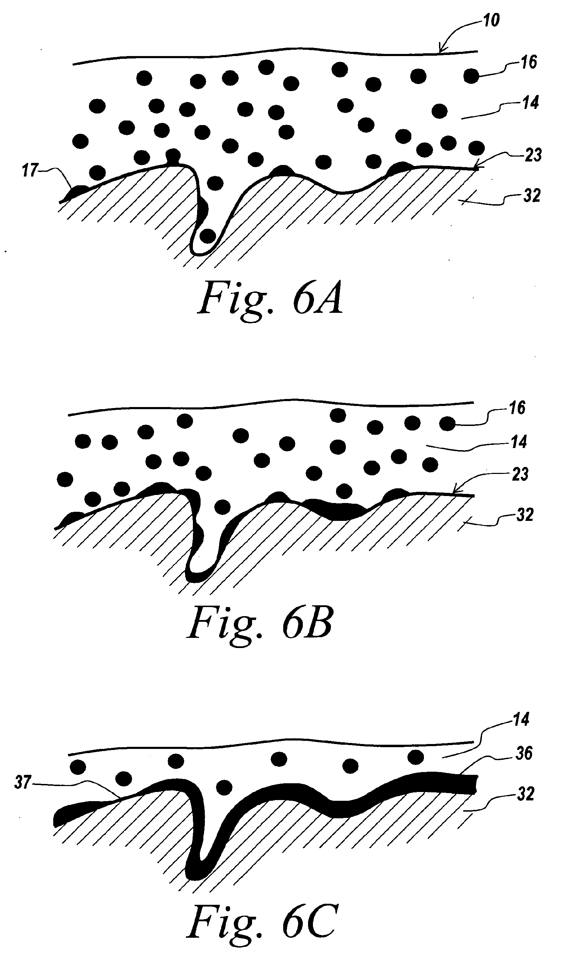

Once the dispersing liquid 10 has been applied to the internal body tissues 32, the dispersing liquid 10 easily flows over surfaces of the internal body tissues 32 and into all of the cracks and crevices in surfaces of the internal body tissues 32 due to the low viscosity of the dispersing liquid 10. **FIG. 6A** illustrates the dispersing liquid 10 immediately after being applied to the internal body tissues 32. The dispersing liquid 10 moves and flows during and after application to the internal body tissues 32. In addition, the droplets of bio-absorbable oil 16 move within the liquid carrier 14. Because of the high density of droplets in the dispersing liquid 10 these movements bring a droplet in contact with a surface 23 of the internal body tissues 22. The droplet adheres to the surface upon contact because the bio-absorbable oil droplets adhere to internal body tissues 32. Subsequently, if a second droplet contacts the first droplet that has adhered to the tissue 17, the second droplet will be less likely to adhere to the first droplet 17 than it would be to adhere to uncoated internal body tissue because the droplets do not preferentially adhere to each other. This action results in the formation of the thin coating of bio-absorbable oil on the tissue.

**FIG. 6B** illustrates the beginning of the formation of a layer of bio-absorbable oil as numerous droplets have adhered to the surface and some begin to coalesce. The energy barrier for two free droplets or one free droplet and one adhered droplet to coalesce is high, but once both droplets have adhered to the surface, the energy barrier for the adhered droplets to coalesce with each other is greatly reduced.

As shown in **FIG. 6C****,** because of the motion (flow) of the dispersing liquid 10 and the motion of the droplets of bio-absorbable oil 16 in the liquid carrier 14, more droplets continue to contact and adhere to surfaces of the internal body tissues 23 until a substantial fraction or portion of the surfaces 23 of the internal body tissues 32 is covered with a bio-absorbable oil layer 36. There may be portions of the surface of the internal body tissues that are not covered by a bio-absorbable oil layer, meaning that the percentage coverage of the surfaces of the internal body tissues by the bio-absorbable oil layer may not be 100%. Ideally, this bio-absorbable oil layer 36 would be complete and uniform, however, the benefits of the bio-absorbable coating layer can be attained with less than 100% coverage of the surfaces of internal body tissues. For example, the minimum percentage coverage required to realize therapeutic benefits depending on the particular treatment can range from about 10% to about 90% for different treatments and for different compositions of the bio-absorbable oil.

Bio-absorbable oils exhibit a temporary and non-tissue reactive adherence effect. The bio-absorbable oil coating adhered to internal tissue may rub off, spread to other tissues in contact with the coating, or adhere to any medical device placed in contact with the coating. These bio-absorbable oils are not surgical adhesives; there is no chemical bond formed for adhesive attachment to the tissue. The bio-absorbable coating layer forms a temporary attachment to the tissue that is non-chemically bonded and non-chemically activated.

The dispersing liquid forms a uniform thin coating of bio-absorbable oil on exposed tissues, implants, and surgical instruments that it contacts. This coating of bio-absorbable oil forms a barrier that hinders air from reaching exposed tissue and that hinders water in the exposed tissue from evaporating into the air.

As discussed above, desiccation of normally moist or wet tissue during surgery can cause serious tissue damage. To prevent desiccation, the dispersing liquid should be applied shortly after the tissue is exposed to air during surgery. The bio-absorbable oil layer will form while the tissue is still wet and prevent the tissue from drying out. As shown in **FIG. 4A****,** the dispersing liquid can be reapplied to the internal body tissues (step 204) throughout surgery to rehydrate tissue and/or to form a bio-absorbable coating on newly exposed tissue. No dose related toxic effects or hypersensitivity are known to occur from the local administration of fish oil when applied locally to tissue, thus a dispersing liquid including fish oil can be reapplied as often as necessary. The dispersing liquid can likewise be applied with a therapeutic agent at instances where the therapeutic agent can be beneficial to the surgical procedure, or to patient healing or comfort.

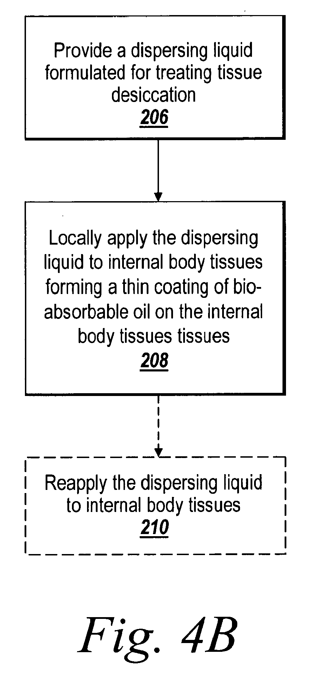

**FIG. 4B** illustrates a method of treating tissue desiccation during surgery. Initially, a dispersing liquid 10 formulated for treating tissue desiccation is provided (step 206). The dispersing liquid 10 includes a suspension 12 including a liquid carrier 14 and a bio-absorbable oil 16 suspended in the liquid carrier 14. The dispersing liquid 10 is locally applied to internal body tissues forming a thin coating of bio-absorbable oil on the internal body tissues (step 208). The liquid carrier 14 can be formulated to rehydrate tissue. Before droplets of bio-absorbable oil 16 adhere to the internal body tissues, the liquid carrier 14 is in liquid contact with surfaces of the internal body tissues. If the internal body tissues are dehydrated before application of the dispersing liquid 10, the liquid carrier 14 can rehydrate the tissue when it is in direct contact with the surfaces of the internal body tissues. After the thin coating of bio-absorbable oil 16 is formed on the internal body tissues, the hydration of the internal body tissues is preserved by the thin coating of bio-absorbable oil inhibiting evaporation of water from the internal body tissues. The dispersing liquid may be reapplied (step 210) as often as necessary to treat tissue desiccation of the internal body tissues.

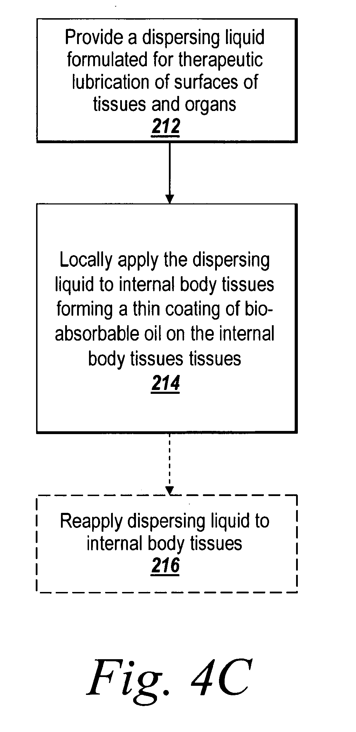

**FIG. 4C** illustrates a method of providing therapeutic lubrication to tissues and organs during and after surgery. Initially, a dispersing liquid 10 formulated for therapeutic lubrication of tissues and organs is provided (step 212). The dispersing liquid 10 includes a suspension 12 which includes a liquid carrier 14 and a bio-absorbable oil 16 suspended in the liquid carrier 14. The dispersing liquid 10 is locally applied to internal body tissues forming a thin coating of bio-absorbable oil on the internal body tissues (step 214).

As discussed above, unwanted tissue adhesion can cause serious tissue trauma and injury. The thin coating of bio-absorbable oil 16 provides lubrication to tissues and organs that reduces tissue adhesion. The thin coating of bio-absorbable oil remains after the conclusion of the surgery to lubricate and prevent tissue adhesion even after a wound is closed. This lubrication can reduce dense connective tissue remodeling after surgery and tissue trauma. The dispersing liquid may be reapplied to internal body tissues (step 216) as often as necessary during surgery to provide therapeutic lubrication.

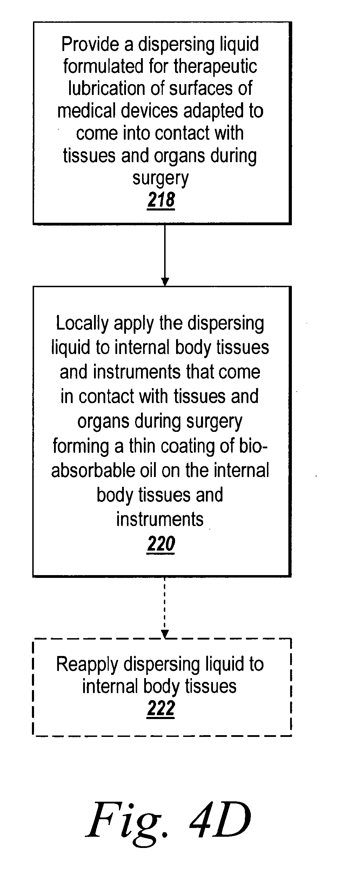

**FIG. 4D** illustrates a method of providing therapeutic lubrication to medical devices adapted to come into contact with tissues and organs during surgery. Initially, a dispersing liquid 10 formulated for therapeutic lubrication of surfaces of medical implants adapted to come into contact with tissues and organs during surgery is provided (step 218). The dispersing liquid 10 includes a suspension 12 which includes a liquid carrier 14 and a bio-absorbable oil 16 suspended in the liquid carrier 14. The dispersing liquid 10 is locally applied to internal body tissues and instruments that come in contact with tissues and organs during surgery forming a thin coating of bio-absorbable oil on the internal body tissues and instruments (step 220). During surgery, the dispersing liquid 10 coats everything that it comes in contact with. Tissues, implants, and surgical tools are coated with bio-absorbable oil 16 lubricant over the areas that the dispersing liquid 10 contacts. The bio-absorbable oil coating acts as a lubricant to prevent adhesion between various tissues, between tissues and implants, and between tissues and surgical instruments. The dispersing liquid may be reapplied (step 222) as often as necessary to provide therapeutic lubrication to surfaces of the medical devices during surgery.

The bio-absorbable oil coating is completely absorbed by the cells of the internal body tissues over time. The thin coating of bio-absorbable oil is typically absorbed over the course of minutes to hours, but can be formulated into a slower uptake material that can take days, weeks, or months; depending upon the make up of the oil chemistry and selected processing of the oil. Because the oil is primarily made up of fatty acid groups which are readily hydrolyzed and broken down into smaller absorbable fatty alcohols, glyricerides and fatty acid components by the cell and tissue, the oil is completely absorbed with no known foreign body response, including no identifiable histological inflammatory response during the absorption period. In short, the coating is generally composed of fatty acids, including in some instances omega-3 fatty acids, bound to glycerol to form mono, di and triglycerides, potentially also including a mixture of free fatty acids and vitamin E. The triglycerides are broken down by lipases (enzymes) which result in free fatty acids that can than be transported across cell membranes. Subsequently, fatty acid metabolism by the cell occurs to metabolize any substances originating with the coating. The bio-absorbable nature of the coating thus results in the coating being absorbed, leaving no by-products. In contrast, bio-degradable lubricant coating would break down into inflammatory by-products with said by-products being dispersed for ultimate elimination by the patient's body.

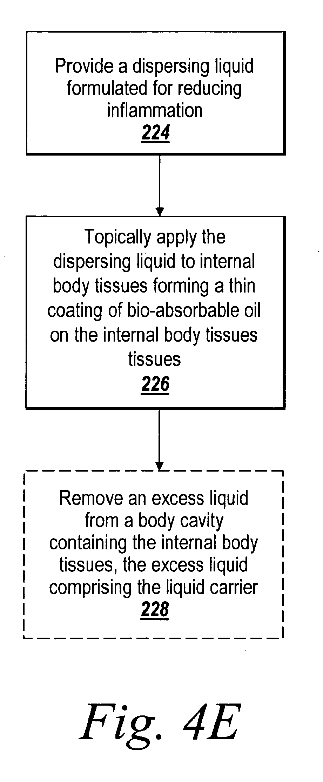

**FIG. 4E** illustrates a method of reducing inflammation. Initially, a dispersing liquid 10 formulated for reducing inflammation is provided (step 224). The dispersing liquid 10 includes a suspension 12 which includes a liquid carrier 14 and a bio-absorbable oil 16 suspended in the liquid carrier 14. The dispersing liquid 10 is topically applied to internal body tissues forming a thin coating of bio-absorbable oil on the internal body tissues (step 226).

The resulting bio-absorbable oil coating covering the internal tissues reduces inflammation directly and indirectly. As discussed above, the bio-absorbable oil coating reduces tissue desiccation and reduces unwanted tissue adhesion, which reduce tissue trauma. This reduction in tissue trauma due to the coating, results in reduced inflammation. Additionally, the bio-absorbable oil itself has anti-inflammatory properties.

According to aspects of the invention, in the methods illustrated by **FIGS. 4A** **- 4E,** the dispersing liquid can include a therapeutic agent and/or an analgesic as described above. The therapeutic agent and/or analgesic may be in the form of a solid, a gel or a liquid. The therapeutic agent and/or analgesic can be included in the liquid carrier, included in the bio-absorbable oil or can be mixed with both after the suspension is formed depending on the particular agent and the desired result. The therapeutic agent and/or analgesic is distributed to the internal body tissues as part of the dispersing liquid. The therapeutic agent and/ or analgesic is incorporated into the bio-absorbable oil coating and so adheres to the internal body tissues. The therapeutic agent and/or analgesic remains in the coating after the surgery and is incorporated into the cells of the internal body tissue gradually as the bio-absorbable coating is absorbed. Incorporating a therapeutic agent and/or analgesic into the bio-absorbable oil coating allows targeted local delivery of the therapeutic agent and/or analgesic to the location of the tissue trauma.

Additionally, the bio-absorbable coating may allow a therapeutic agent and/or analgesic to more easily enter tissue cells. The bio-absorbable coating contains lipids, many of which originate as triglycerides. It has previously been demonstrated that triglyceride products such as partially hydrolyzed triglycerides and fatty acid molecules can integrate into cellular membranes and enhance the solubility of drugs into the cell. Whole triglycerides are known not to enhance cellular uptake as well as partially hydrolyzed triglyceride, because it is difficult for whole triglycerides to cross cell membranes due to their relatively larger molecular size. The bio-absorbable oil coating facilitates effective topical treatment with a therapeutic agent and/or analgesic.

If the dispersing liquid 10 includes a topical analgesic to reduce localized pain, the analgesic compound is mixed into the dispersing liquid 10 just prior to application to the internal body tissues to maximize the amount of time that the analgesic is effective in the body. The half life of a water soluble analgesic like liquid Bupivacaine HCL (without epinephrine) is 2-3 hours. The dispersing liquid including the analgesic can be reapplied throughout surgery and just before closing the wound in order to reduce pain.

Chemically different oil blends such as vitamin E and its variants may be used to alter the viscosity of the bio-absorbable oil for more uniform distribution or to act as a solvent for therapeutic agents and/or analgesics included in the dispersing liquid. Chemically different oil blends can also be used to improve coating adherence to the body tissues or to controllably affect the rate of separation of the emulsion.

Vitamin E compounds can also integrate into cellular membranes resulting in decreased membrane fluidity and cellular uptake. It should be noted that as utilized herein to describe the present invention, the term vitamin E and the term alpha-tocopherol, are intended to refer to the same or substantially similar substance, such that they are interchangeable and the use of one includes an implicit reference to both. Further included in association with the term vitamin E are such variations including but not limited to one or more of alpha-tocopherol, beta-tocopherol, delta-tocopherol, gamma-tocopherol, alpha-tocotrienol, beta-tocotrienol, delta-tocotrienol, gamma-tocotrienol, alpha-tocopherol acetate, beta-tocopherol acetate, gamma-tocopherol acetate, delta-tocopherol acetate, alpha-tocotrienol acetate, beta-tocotrienol acetate, delta-tocotrienol acetate, gamma-tocotrienol acetate, alpha-tocopherol succinate, beta-tocopherol succinate, gamma-tocopherol succinate, delta-tocopherol succinate, alpha-tocotrienol succinate, beta-tocotrienol succinate, delta-tocotrienol succinate, gamma-tocotrienol succinate, mixed tocopherols, vitamin E TPGS, derivatives, analogs and pharmaceutically acceptable salts thereof. It should also be noted that other antioxidants can be used as a substitute to fulfill the functions of Vitamin E in this coating.

The methods illustrated by **FIGS. 4A-4E****,** may result in excess carrier liquid and excess bio-absorbable oil pooled in a body cavity containing the internal body tissue after the formation of the bio-absorbable coating. This excess fluid is formulated or configured to be excreted by the body or absorbed by the body over time. The rate of excretion and absorption of the excess carrier liquid and excess bio-absorbable oil may be different than the rate of absorption of the bio-absorbable oil coating that adheres to the internal body tissues. Excess carrier liquid and bio-absorbable oil can likewise be manually evacuated from the body cavity during surgery after the bio-absorbable oil coating has been formed as illustrated in step 228 of **FIG. 4E****.** Methods to evacuate the excess liquid include removal by a vacuum assist device, gravity evacuation using a closed wound catheter drainage device, or physical removal by absorbent materials applied to the collected fluid. At the time that the fluid is evacuated, it may be fully separated into liquid carrier and bio-absorbable oil components, it may be in the process of separating, or it may still contain a significant quantity of bio-absorbable oil dispersed in the liquid carrier. When the excess liquid is removed from the body cavity, the thin coating of bio-absorbable oil remains on the internal body tissues.

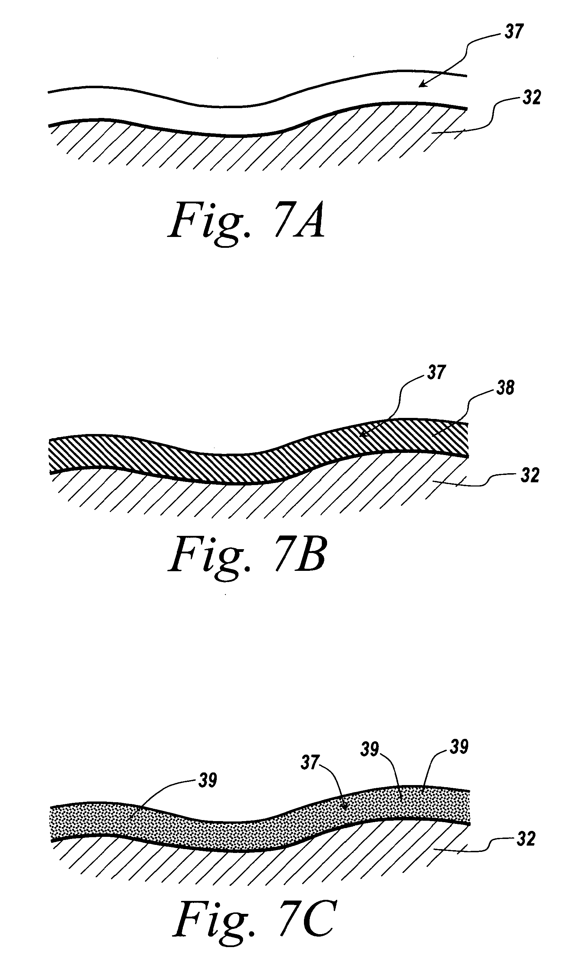

In **FIGS. 6A, 6B** and **6C** the bio-absorbable oil 16 is shown with a dark color for clarity, however, may bio-absorbable oils are light in color or colorless making a thin coating of bio-absorbable oil on internal body tissues difficult to see. **FIG. 7A** illustrates the bio-absorbable oil coating 37 on internal body tissues 32 which includes no dyes, visibly detectable color additives or imaging enhancing agents. According to aspects of an exemplary embodiment, the dispersing liquid 10 can additionally include visibly detectable color additives 38 or imaging enhancing agents 39. **FIG. 7B** illustrates the bio-absorbable oil coating including a visibly detectable color additive 38. Visibly detectable color additives 38 incorporated into the bio-absorbable oil coating 37 allow a surgeon to verify that the bio-absorbable oil coating 37 has formed and that the coating covers the internal body tissues 32. It also allows a surgeon to visually determine when reapplication of the dispersing liquid 10 is necessary.

**FIG. 7C** illustrates the bio-absorbable oil coating including a particle imaging enhancing agent 39. Liquid, bubble or particle imaging enhancing agents (i.e. radiopaque x-ray imaging dye) for ultrasound imaging enhancement, positron emission tomography (PET) scanning, computed tomography (CT) scanning, x-ray or magnetic resonance (MR) imaging of body tissues can be incorporated into the bio-absorbable oil coating to allow easier imaging of surfaces of internal body tissues 32. Additionally, the imaging enhancing agent 39 can be used to improve specific targeted radiation treatment of internal body tissues 32.

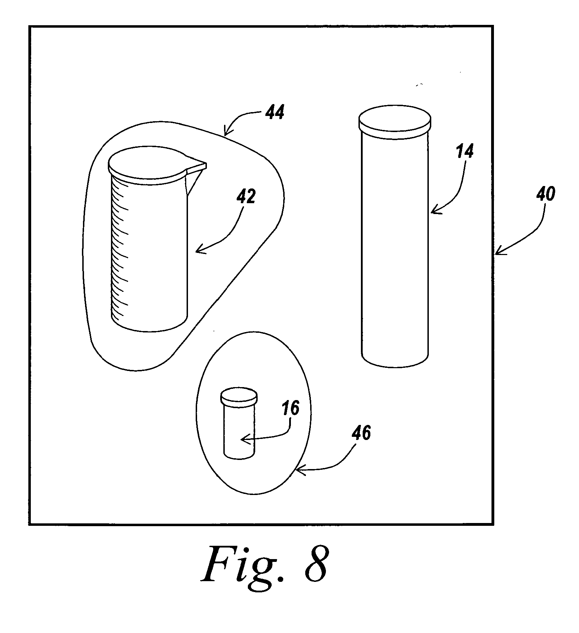

**FIG. 8** illustrates another exemplary embodiment of the invention which includes a kit 40 for topically coating internal body tissues with a coating emulsion. The kit 40 includes a liquid carrier 14 formulated for local application to internal body tissues and a bio-absorbable oil 16 formulated for local application to internal body tissues. The kit 40 can also include a sterile mixing apparatus 42. The mixing apparatus 42 can be sealed in a sterile package 44. The bio-absorbable oil can be contained within the same sterile packaging 44 or the bio-absorbable oil can be contained within separate sterile packaging 46. The sterile mixing apparatus 42 can be closeable or sealable to allow manual mixing or agitation of the liquid carrier 14, bio-absorbable oil 16 and any additional components to form an emulsion 12 shortly before application to the internal body tissues. The sterile mixing apparatus 42 can be configured to allow easy measurements of volume for adding specific quantities (dose control) of therapeutic agents, dilution adjustment capability of the emulsion and measurement or control of the carrier liquid to bio-absorbable oil ratio. The sterile mixing apparatus 42 can be sized and configured to minimize the exposure of the dispersing liquid 10 to air prior to application. The sterile mixing apparatus 42 can be configured to minimize liquid volume loss when liquid applicators are used to remove the dispersing liquid 10 from the apparatus for application to the internal body tissues 32. Alternately, the sterile mixing apparatus 42 itself can be configured to apply the dispersing liquid 10 to the internal body tissues 32. The kit 40 can further include an applicator. Although FIG. 8 depicts an embodiment in which the liquid carrier 14 and the bio-absorbable oil 46 are stored in separate containers, the liquid carrier 14 and the bio-absorbable oil 46 can be stored in the same container. The liquid carrier 14 and the bio-absorbable oil can be premeasured so that measuring is not required.

As described herein, the present invention relates to a dispersing liquid for coating internal body tissues including a suspension of a bio-absorbable oil suspended in a liquid carrier, a method of making the dispersing liquid, and a kit for coating internal body tissues with the dispersing liquid. Upon application, the liquid carrier disperses the suspended bio-absorbable oil droplets and the bio-absorbable oil droplets adhere to surfaces of the internal body tissues forming a thin coating of bio-absorbable oil. This coating prevents desiccation of normally wet tissues during surgery and provides therapeutic lubrication to reduce unwanted tissue adhesion and reduces inflammation due to tissue trauma. The bio-absorbable oil coating has anti-inflammatory properties. Additional therapeutic agents and topical analgesics can be included in the bio-absorbable coating. The bio-absorbable oil coating forms a non-chemically bonded and non-chemically active adhesion to the internal body tissue. The coating is fully absorbed by the cells of the internal body tissues over time leaving no byproducts of breakdown to cause inflammation.

## Claims

1. A dispersing liquid for distributing a bio-absorbable oil to internal body tissues, comprising:
a suspension formulated for local application to internal body tissues, comprising:
a liquid carrier; and
a bio-absorbable oil suspended in the liquid carrier,
and wherein the suspension comprises droplets of bio-absorbable oil suspended in the liquid carrier, wherein the droplets of oil adhere to the internal body tissue and coalesce forming a thin coating of bio-absorbable oil at least partially covering the tissues, and wherein the suspension is not stabilized.

2. The dispersing liquid of claim 1, wherein the bio-absorbable oil comprises at least one form of lipid.

3. The dispersing liquid of claim 1, wherein the bio-absorbable oil comprises at least one form of fatty acid.

4. The dispersing liquid of claim 1 wherein the bio-absorbable oil comprises one or both of a synthetic bio-absorbable oil or a naturally occurring bio-absorbable oil.

5. The dispersing liquid of claim 1 or claim 2, wherein the bio-absorbable oil is configured to adhere to internal body tissues upon application of the suspension to internal body tissues.

6. The dispersing liquid of claim 1 wherein the liquid carrier comprises at least one liquid carrier selected from a group of liquid carriers comprising: water, an alcohol, a solution of dextrose in water, a sodium phosphate solution, a hydrochloric acid solution, and a saline solution.

7. The dispersing liquid of claim 1 wherein the bio-absorbable oil comprises a combination of a plurality of types of bio-absorbable oils.

8. The dispersing liquid of claim 1 further comprising a therapeutic agent.

9. The dispersing liquid of claim 8, wherein the therapeutic agent is in the form of a solid suspended in the liquid carrier, a solid suspended in the bio-absorbable oil, or both.

10. The dispersing liquid of claim 1 further comprising one or more of an anti-proliferative agent, an anti-inflammatory agent, or an anti-infective agent

11. The dispersing liquid of claim 1 further comprising a local analgesic agent.

12. The dispersing liquid of claim 1 further comprising one or both of an imaging enhancing agent or a visibly detectable color additive.

13. A method for making a dispersing liquid, the method comprising:
providing a bio-absorbable oil;
and wherein the suspension comprises droplets of bio-absorbable oil suspended in the liquid carrier, wherein the droplets of oil adhere to the internal body tissue and coalesce forming a thin coating of bio-absorbable oil at least partially covering the tissues, and wherein the suspension is not stabilized.
providing a liquid carrier; and
mixing the bio-absorbable oil and the liquid carrier to form a suspension of liquid bio-absorbable oil suspended in the liquid carrier and formulated for local application to internal body tissues.

14. The method of claim 13, wherein the suspension is not stabilized.

15. A kit for coating internal body tissues with a coating suspension comprising:
a liquid carrier suitable for local application to internal body tissues; and
a bio-absorbable oil suitable for local application to internal body tissues,
and wherein the suspension comprises droplets of bio-absorbable oil suspended in the liquid carrier, wherein the droplets of oil adhere to the internal body tissue and coalesce forming a thin coating of bio-absorbable oil at least partially covering the tissues, and wherein the suspension is not stabilized.

16. The kit of claim 15 further comprising a sterile measured mixing apparatus.

17. The kit of claim 16, wherein the sterile measured mixing apparatus is an applicator.

18. The kit of claim 16 further comprising packaging to maintain the sterility of the measured mixing apparatus.

19. The kit of claim 15, further comprising an applicator.

## Patentansprüche

1. Dispergierflüssigkeit zum Verteilen eines biologisch resorbierbaren Öls an innere Körpergewebe, umfassend:
eine Suspension, die zur lokalen Auftragung auf innere Körpergewebe formuliert ist, umfassend:
einen flüssigen Träger und
ein biologisch resorbierbares Öl, das in dem flüssigen Träger suspendiert ist,
und wobei die Suspension Tröpfchen von biologisch resorbierbarem Öl umfasst, die in dem flüssigen Träger suspendiert sind, wobei die Öltröpfchen an dem inneren Körpergewebe haften und koaleszieren, um eine dünne Beschichtung des biologisch resorbierbaren Öls zu bilden, die die Gewebe mindestens teilweise bedeckt, und wobei die Suspension nicht stabilisiert ist.

2. Dispergierflüssigkeit nach Anspruch 1, wobei das biologisch resorbierbare Öl mindestens eine Form von Lipid umfasst.

3. Dispergierflüssigkeit nach Anspruch 1, wobei das biologisch resorbierbare Öl mindestens eine Form von Fettsäure umfasst.

4. Dispergierflüssigkeit nach Anspruch 1, wobei das biologisch resorbierbare Öl eines oder beide von einem synthetischen biologisch resorbierbaren Öl oder einem natürlich vorkommenden biologisch resorbierbaren Öl umfasst.

5. Dispergierflüssigkeit nach Anspruch 1 oder Anspruch 2, wobei das biologisch resorbierbare Öl so ausgestaltet ist, dass es bei Auftragung der Suspension auf interne Körpergewebe an den internen Körpergeweben haftet.

6. Dispergierflüssigkeit nach Anspruch 1, wobei der flüssige Träger mindestens einen flüssigen Träger ausgewählt aus einer Gruppe von flüssigen Trägern umfasst, die Wasser, einen Alkohol, eine Lösung von Dextrose in Wasser, eine Natriumphosphatlösung, eine Salzsäurelösung und eine Kochsalzlösung umfasst.

7. Dispergierflüssigkeit nach Anspruch 1, wobei das biologisch resorbierbare Öl eine Kombination einer Vielzahl von Typen von biologisch resorbierbaren Ölen umfasst.

8. Dispergierflüssigkeit nach Anspruch 1, die ferner ein therapeutisches Mittel umfasst.

9. Dispergierflüssigkeit nach Anspruch 8, wobei das therapeutische Mittel in Form eines Feststoffs, der in dem flüssigen Träger suspendiert ist, eines Feststoffs, der in dem biologisch resorbierbaren Öl suspendiert ist, oder beiden vorliegt.

10. Dispergierflüssigkeit nach Anspruch 1, die ferner ein oder mehrere von einem antiproliferativen Mitteln, einem antientzündlichen Mittel oder einem antünfektiven Mittel umfasst.

11. Dispergierflüssigkeit nach Anspruch 1, die ferner ein Lokalanästhetikum umfasst.

12. Dispergierflüssigkeit nach Anspruch 1, die ferner eines oder beide von einem Bildgebungsverstärkungsmittel oder einem sichtbar detektierbaren Farbadditiv umfasst.

13. Verfahren zum Herstellen einer Dispergierflüssigkeit, wobei das Verfahren umfasst:
Bereitstellen eines biologisch resorbierbaren Öls;
und wobei die Suspension Tröpfchen von biologisch resorbierbarem Öl umfasst, die in dem flüssigen Träger suspendiert sind, wobei die Öltröpfchen an dem inneren Körpergewebe haften und koaleszieren, um eine dünne Beschichtung des biologisch resorbierbaren Öls zu bilden, die die Gewebe mindestens teilweise bedeckt, und wobei die Suspension nicht stabilisiert ist, Bereitstellen eines flüssigen Trägers und
Mischen des biologisch resorbierbaren Öls und des flüssigen Trägers, um eine Suspension des flüssigen biologisch resorbierbaren Öls zu bilden, die in dem flüssigen Träger suspendiert ist und zur lokalen Auftragung auf interne Körpergewebe formuliert ist.

14. Verfahren nach Anspruch 13, wobei die Suspension nicht stabilisiert ist.

15. Kit zum Beschichten interner Körpergewebe mit einer Beschichtungssuspension, umfassend:
einen flüssigen Träger, der zur lokalen Auftragung auf innere Körpergewebe geeignet ist, und
ein biologisch resorbierbares Öl, das zur lokalen Auftragung auf innere Körpergewebe geeignet ist,
und wobei die Suspension Tröpfchen von biologisch resorbierbarem Öl umfasst, die in dem flüssigen Träger suspendiert sind, wobei die Öltröpfchen an dem inneren Körpergewebe haften und koaleszieren, um eine dünne Beschichtung des biologisch resorbierbaren Öls zu bilden, die die Gewebe mindestens teilweise bedeckt, und wobei die Suspension nicht stabilisiert ist.

16. Kit nach Anspruch 15, das ferner einen sterilen abgemessenen Mischapparat umfasst.

17. Kit nach Anspruch 16, wobei der sterile abgemessene Mischapparat ein Applikator ist.

18. Kit nach Anspruch 16, das ferner Verpackung umfasst, um die Sterilität des abgemessenen Mischapparats aufrechtzuerhalten.

19. Kit nach Anspruch 15, das ferner einen Applikator umfasst.

## Revendications

1. Liquide de dispersion pour distribuer une huile bioabsorbable aux tissus corporels internes, comprenant :
une suspension formulée pour l'application locale aux tissus corporels internes, comprenant :
un support liquide ; et
une huile bioabsorbable en suspension dans le
support liquide,
et dans lequel la suspension comprend des gouttelettes d'huile bioabsorbable en suspension dans le support liquide, dans lequel les gouttelettes d'huile adhèrent aux tissus corporels internes et se coalescent pour former un revêtement mince d'huile bioabsorbable recouvrant au moins partiellement les tissus, et dans lequel la suspension n'est pas stabilisée.

2. Liquide de dispersion selon la revendication 1, dans lequel l'huile bioabsorbable comprend au moins une forme de lipide.

3. Liquide de dispersion selon la revendication 1, dans lequel l'huile bioabsorbable comprend au moins une forme d'acide gras.

4. Liquide de dispersion selon la revendication 1, dans lequel l'huile bioabsorbable comprend l'un des deux ou les deux parmi une huile bioabsorbable synthétique ou une huile bioabsorbable d'origine naturelle.

5. Liquide de dispersion selon la revendication 1 ou 2, dans lequel l'huile bioabsorbable est configurée pour adhérer aux tissus corporels internes dès l'application de la suspension aux tissus corporels internes.

6. Liquide de dispersion selon la revendication 1, dans lequel le support liquide comprend au moins un support liquide choisi dans un groupe de supports liquides comprenant : l'eau, un alcool, une solution de dextrose dans l'eau, une solution de phosphate de sodium, une solution d'acide chlorhydrique, et une solution saline.

7. Liquide de dispersion selon la revendication 1, dans lequel l'huile bioabsorbable comprend une combinaison d'une pluralité de types d'huiles bioabsorbables.

8. Liquide de dispersion selon la revendication 1, comprenant en outre un agent thérapeutique.

9. Liquide de dispersion selon la revendication 8, dans lequel l'agent thérapeutique est sous forme d'un solide en suspension dans le support liquide, d'un solide en suspension dans l'huile bioabsorbable, ou les deux.

10. Liquide de dispersion selon la revendication 1, comprenant en outre un ou plusieurs parmi un agent antiprolifératif, un agent anti-inflammatoire, ou un agent anti-infectieux.

11. Liquide de dispersion selon la revendication 1, comprenant en outre un agent analgésique local.

12. Liquide de dispersion selon la revendication 1, comprenant en outre l'un des deux ou les deux parmi un agent améliorant l'image ou un additif coloré détectable visuellement.

13. Procédé de préparation d'un liquide de dispersion, le procédé comprenant :
la fourniture d'une huile bioabsorbable ;
et dans lequel la suspension comprend des gouttelettes d'huile bioabsorbable en suspension dans le support liquide, dans lequel les gouttelettes d'huile adhèrent aux tissus corporels internes et se coalescent pour former un revêtement mince d'huile bioabsorbable recouvrant au moins partiellement les tissus, et dans lequel la suspension n'est pas stabilisée ;
la fourniture d'un support liquide ; et
le mélange de l'huile bioabsorbable et du support liquide pour former une suspension d'huile bioabsorbable liquide en suspension dans le support liquide et formulée pour l'application locale aux tissus corporels internes.

14. Procédé selon la revendication 13, dans lequel la suspension n'est pas stabilisée.

15. Kit pour le revêtement de tissus corporels internes avec une suspension de revêtement comprenant :
un support liquide adapté à l'application locale aux tissus corporels internes ; et
une huile bioabsorbable adaptée à l'application locale aux tissus corporels internes,
et dans lequel la suspension comprend des gouttelettes d'huile bioabsorbable en suspension dans le support liquide, dans lequel les gouttelettes d'huile adhèrent aux tissus corporels internes et se coalescent pour former un revêtement mince d'huile bioabsorbable recouvrant au moins partiellement les tissus, et dans lequel la suspension n'est pas stabilisée.

16. Kit selon la revendication 15, comprenant en outre un appareil de mélange mesuré stérile.

17. Kit selon la revendication 16, dans lequel l'appareil de mélange mesuré stérile est un applicateur.

18. Kit selon la revendication 16, comprenant en outre l'emballage pour maintenir la stérilité de l'appareil de mélange mesuré.

19. Kit selon la revendication 15, comprenant en outre un applicateur.
